# FASCICULE DE BREVET EUROPEEN

(11) **EP 1 243 252 B1**
(45) Date de publication et mention de la délivrance du brevet: **24.05.2006**
(21) Numéro de dépôt: 02290636.6
(22) Date de dépôt: 13.03.2002
(51) Int. Cl.: A61Q 19/08

(54) **Composition pour le peau, contenant des fibres et des ubiquinones**
Hautbehandlungsmittel enthaltend Fasern und Ubichinone
Skin composition containing fibres and ubiquinones

(30) Priorité: 23.03.2001 FR 0103960
(43) Date de publication de la demande: 25.09.2002
(73) Titulaire: L'OREAL, 75008 Paris (FR)
(72) Inventeur: Chevalier, Veronique, 94440 Villecresnes (FR)
(74) Mandataire: Rasson, Catherine

(56) Documents cités:
- EP-A- 1 053 742
- EP-A- 1 064 930
- EP-A- 1 172 078

## Description

La présente invention a pour objet une composition pour application topique sur la peau, contenant des fibres et au moins un ou plusieurs composés choisis dans le groupe des ubiquinones et de leurs dérivés, et aux utilisations de ladite composition, notamment dans les domaines cosmétique et dermatologique.

Il est connu d'utiliser dans les compositions cosmétiques, notamment des ubiquinones, par exemple pour traiter les signes du vieillissement de la peau humaine.

On sait qu'au cours du processus de vieillissement, il apparaît différents signes sur la peau humaine, très caractéristiques de ce vieillissement, se traduisant notamment par une modification de la structure et de la fonction cutanée. Les principaux signes cliniques de vieillissement cutané sont notamment les suivants : apparition de ridules puis de rides profondes en augmentation avec l'âge, et désorganisation du "grain" de la peau, c'est-à-dire que le micro-relief est moins régulier et présente un caractère anisotrope.

Par ailleurs, le teint de la peau est généralement modifié ; il apparaît plus pâle et plus jaune, ce qui semble être dû essentiellement à une désorganisation de la microcirculation (moins d'hémoglobine au niveau du derme papillaire). De nombreuses taches colorées apparaissent en surface, ce qui est dû à une mélanogénèse altérée. Un autre signe clinique de vieillissement est l'aspect sec et rêche de la peau qui est dû essentiellement à une desquamation plus importante, ces squames en diffractant les rayons lumineux participant aussi à l'aspect un peu gris du teint.

Ainsi, les ubiquinones sont utilisées pour traiter certains de ces signes du vieillissement, par exemple pour traiter ou prévenir la sécheresse de la peau, la couperose, le vieillissement chronologique, l'aspect rêche de la peau, pour traiter les rides et ridules et/ou les taches cutanées.

Par ailleurs, les ubiquinones sont utilisées pour éviter le regraissage de la peau et/ou des cheveux après lavage, pour nourrir la peau et lutter contre sa sécheresse, pour traiter l'acné et traiter les blessures.

Toutefois, ces actifs présentent l'inconvénient de n'être efficaces qu'après un certain temps d'application. Or, on cherche de plus en plus à obtenir un effet immédiat des actifs utilisés, conduisant rapidement à un lissage des rides et ridules, à la disparition des marques de fatigue de la peau, à l'estompage des signes apparents du vieillissement, afin d'obtenir une peau lisse et qui respire la bonne santé.

La demanderesse a trouvé de manière surprenante que l'association de fibres et d'ubiquinone permettait d'obtenir à la fois un effet immédiat et un effet à long terme sur les signes visibles du vieillissement (ridules, rides, taches, teint terne) et de toute autre imperfection de la matière kératinique traitée et notamment de la peau, tout en gardant une apparence naturelle à la peau. En outre, les fibres permettent d'apporter de bonnes propriétés cosmétiques : douceur et confort lors de l'application sur la peau, facilité d'application.

Le document EP-A-1172078 qui constitue un art antérieur à la présente demande selon l'article 54(3)CBE décrit des mascaras contenant des fibres élastomères.

Des compositions de soin ou de maquillage contenant des fibres sont connues des documents EP-A-1064930 et EP-A-1053742.

La présente invention a pour objet une composition pour application topique sur la peau, contenant des fibres et au moins un ou plusieurs composés choisis dans le groupe des ubiquinones et de leurs dérivés.

On entend ici par « application topique » une application externe sur les matières kératiniques, et par « matières kératiniques », on entend notamment la peau y compris le cuir chevelu, et les muqueuses (lèvres). La composition peut être notamment une composition cosmétique ou dermatologique pour la peau et les lèvres.

La composition selon l'invention étant destinée à une application topique comprend un milieu physiologiquement acceptable, c'est-à-dire compatible avec toutes les matières kératiniques telles que la peau du visage (front, joues, contour des lèvres), des mains et du corps y compris le cuir chevelu, et les lèvres.

Les ubiquinones sont des dérivés quinoniques comportant ou non des chaînes latérales isoprène. Elles sont décrites dans le Merck Index, 11^{ème} édition, Merc & Co., Inc. Rahway, N.Y., USA, Abstr. 9751 (1989). Celles comportant une chaîne latérale isoprène sont désignées aussi sous le terme de Co-enzymes Q. Dans les Co-enzymes Q, le nombre d'unités isoprène dans la chaîne latérale est donné par le nombre n qui accompagne « Q » dans la désignation de Co-enzyme Qn. n est un nombre entier allant de 0 à 12, en particulier de 1 à 12 et plus particulièrement de 6 à 10. L'ubiquinone peut aussi ne pas comporter de chaîne latérale isoprène et être choisie parmi les alkyl-ubiquinones où le groupe alkyle peut comporter de 1 à 20 et de préférence de 1 à 12 atomes de carbone, telles que par exemple les décyl-ubiquinones comme la 6-décylubiquinone ou la 2,3-diméthoxy-5-décyl-1,4-ubiquinone, leurs dérivés, et leurs mélanges.

Selon un mode préféré de réalisation de l'invention, l'ubiquinone utilisée dans la composition de l'invention est la co-enzyme Q10.

La composition de l'invention peut comprendre un ou plusieurs ubiquinones ou dérivés d'ubiquinones décrits ci-dessus de la même catégorie ou de différentes catégories. La quantité d'ubiquinone(s) dans la composition dépend de l'ubiquinone utilisée et de la finalité de la composition. La ou les ubiquinones doivent être en quantité efficace, c'est-à-dire en quantité suffisante pour atteindre le but recherche qui est l'amélioration de l'apparence de la peau et le traitement de la peau. Cette quantité peut aller par exemple de 0,0001 à 30 % en poids, de préférence de 0,01 à 20 % en poids, mieux de 0,1 à 15 % en poids et encore mieux de 0,5 à 10 % en poids de matière active par rapport au poids total de la composition.

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 µm (0,001 mm) à 10 mm, de préférence de 0,1 µm à 5 mm et mieux de 0,1 mm à 1,5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles. Elles sont choisies parmi les fibres de soie, de coton, de laine, de lin, de cellulose extraites notamment du bois, des légumes ou des algues, de polyamide (notamment de Nylon® ), de cellulose modifiée (rayonne, viscose, acétate notamment d'acétate de rayonne), de poly-p-phénylène téréphtalamide notamment de Kevlar® , en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

Comme fibres de polyuréthane, on peut citer les fibres en polymère poly(uréthane-urée) segmenté, appartenant à la classe des élastanes, et notamment celles commercialisées sous le nom Lycra® par la société Du PONT.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

On peut aussi utiliser des mélanges des fibres citées ci-dessus.

Par ailleurs, les fibres peuvent être traitées ou non en surface, enrobées ou non. Il peut s'agir notamment de fibres enrobées et/ou fonctionnalisées, « fonctionnalisées » signifiant que les fibres sont traitées en surface afin d'en modifier les propriétés.

Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres peuvent être également fonctionnalisées, c'est-à-dire être modifiées de manière à avoir une fonction spécifique. Cette fonctionnalisation des fibres peut être réalisée aussi bien sur les fibres que dans les fibres, et ce par toute méthode permettant d'accrocher un composé aux fibres ou de le piéger dans les cavités formées par la géométrie des fibres. Comme méthodes, on peut citer par exemple l'enduction des fibres par un actif ; la fixation de particules renfermant un actif, telles que nanocapsules ou nanosphères, sur les fibres ; l'adsorption dans les fibres ; la fixation par réaction chimique. On peut ainsi utiliser des fibres ayant des fonctionnalités particulières, par exemple des fibres anti-UV par modification avec filtres solaires chimiques ou physiques; des fibres bactéricides ou antiseptiques par modification avec des conservateurs ou d'anti-bactériens ; des fibres colorées par modification avec des molécules colorantes ; des fibres kératolytiques ou desquamantes par modification avec des agents kératolytiques ou desquamants ; des fibres hydratantes par modification avec des agents hydratants ou des polymères rétenteurs d'eau ; les fibres parfumées par modification avec un parfum ; des fibres analgésiques ou apaisantes par modification avec un anti-inflammatoire ou un agent apaisant ; des fibres anti-transpirantes par modification avec un anti-transpirant.

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-p-phénylène téréphtalamide, les fibres de coton et leurs mélanges. Leur longueur peut aller de 0,1 à 10 mm, de préférence de 0,1 à 1 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut aussi utiliser des fibres de coton par exemple celles qui ont un diamètre moyen de 20 µm, une longueur de 0,3 mm, et un facteur de forme de 15, telles que celles commercialisées par la société Filature de Lomme, par la société Textiles des Dunes, l'Institut Textile de France, ou par la société Velifil.

Les fibres peuvent être présentes dans la composition selon l'invention en une quantité allant par exemple de 0,01 à 50 % en poids, de préférence de 1 à 20 % en poids et mieux de 5 à 10 % en poids de matière active par rapport au poids total de la composition.

Le milieu physiologiquement acceptable des compositions à application topique, selon l'invention peut plus particulièrement comprendre de l'eau et éventuellement un solvant organique physiologiquement acceptable choisi par exemple parmi les alcools inférieurs comportant de 1 à 8 atomes de carbone et de préférence 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol.

Ce milieu peut être aussi un milieu anhydre, notamment un milieu huileux contenant des huiles et/ou matières grasses autres que les huiles.

Selon un mode préféré de réalisation de l'invention, le milieu de la composition comprend de l'eau. Ce milieu aqueux a de préférence un pH compatible avec la peau, allant de préférence de 3 à 8 et mieux de 4,5 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

La phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1® " et "FLUTEC PC3® " par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050® " et "PF 5060® " par la Société 3M, ou encore le bromoperfluorooctyle vendu sous la dénomination "FORALKYL® " par la Société Atochem; le nonafluorométhoxybutane vendu sous la dénomination "MSX 4518® " par la Société 3M et le nonafluoroéthoxyisobutane ; les dérivés de perfluoromorpholine, tels que la 4-trifluorométhyl perfluoromorpholine vendue sous la dénomination "PF 5052® " par la Société 3M ;
- les huiles de silicone comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les compositions selon l'invention peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple (E/H/E ou H/E/H), de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Selon un mode particulier de réalisation de l'invention, la composition selon l'invention est une émulsion. La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Quand l'émulsion est multiple, elle comporte généralement un tensioactif dans l'émulsion primaire et un tensioactif dans la phase externe dans laquelle est introduite l'émulsion primaire.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de Polyglyceryl-4 isostearate/Cetyl dimethicone copolyol/Hexyl laurate vendu sous la dénomination « Abil WE 09 » par la société Goldschmidt, le mélange de cyclomethicone et de dimethicone copolyol, vendu sous les dénominations « DC 5225 C » et « DC 3225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose, et leurs mélanges comme par exemple le mélange de stéarate de glyceryle et de stearate de PEG-100, commercialisé sous la dénomination Arlacel 165 par la société Uniqema.

On peut aussi préparer des émulsions sans tensioactifs en utilisant des composés appropriés, par exemple les polymères ayant des propriétés émulsionnantes tels que le carbopol 1342 et les PEMULEN, et les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol ; nom CTFA : Diglycol/CHDM/lsophtalates/SIP Copolymer) vendus sous les dénominations "Eastman AQ polymer" (AQ35S, AQ38S, AQ55S, AQ48 Ultra) par la société Eastman Chemical.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles autres que ceux cités ci-dessus, les conservateurs, les antioxydants, les solvants, les parfums, les charges, les bactéricides, les absorbeurs d'odeur, les matières colorantes, les sels, les polymères (par exemple Acrylates/Dimethicone Copolymer commercialisé sous la dénomination KP-561 par Shin-Etsu, comme dispersant). Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Les compositions de l'invention peuvent contenir notamment un ou plusieurs actifs lipophiles ou hydrophiles autres que les ubiquinones, choisis parmi les agents hydratants, les agents anti-radicaux libres, les agents kératolytiques, les vitamines, les agents anti-élastase et anti-collagénase, les protides, les dérivés d'acides gras, les stéroïdes, les oligo-éléments, les agents blanchissants, les extraits d'algues et de planctons, les filtres, les enzymes, les flavonoïdes, les céramides et leurs mélanges.

Comme agents hydratants, on peut citer en particulier le lactate de sodium ; les polyols, et en particulier la glycérine, le sorbitol, les polyéthylène glycols; le mannitol ; les acides aminés ; l'acide hyaluronique ; la lanoline ; l'urée et les mélanges contenant de l'urée, tels que le NMF (« Natural Moisturising Factor ») ; la vaseline ; et leurs mélanges.

Comme agents kératolytiques, on peut citer par exemple les α-hydroxy-acides notamment les acides dérivés de fruits, comme les acides glycolique, lactique, malique, citrique, tartrique, mandélique et leurs dérivés ; les β-hydroxy-acides comme l'acide salicylique et ses dérivés tels que l'acide n-octanoyl-5-salicylique ou l'acide n-dodécanoyl-5 salicylique; les α-céto-acides comme l'acide ascorbique ou vitamine C et ses dérivés ; les β-céto-acides; les rétinoïdes comme le rétinol (vitamine A) et ses esters, le rétinal, l'acide rétinoïque et ses dérivés, ceux décrits dans les documents FR-A-2 570 377, EP-A-199 636, EP-A-325 540, EP-A-402 072, et leurs mélanges.

Comme vitamines, outre les vitamines A, E et C indiquées ci-dessus, on peut citer en particulier la vitamine B3 (ou vitamine PP ou niacinamide), la vitamine B5 (panthénol), les dérivés et précurseurs de ces vitamines ainsi que ceux des vitamines A, E et C, comme les lycopènes ou les carotènes précurseurs de la vitamine A, et leurs mélanges.

Comme agents anti-radicaux libres, on peut citer notamment les dérivés de l'acide phosphonique ; l'acide éthylènediamine tétracétique et ses sels tels que le sel de sodium ; la guanosine ; la superoxydismutase ; le tocophérol (vitamine E) et ses dérivés (acétate) ; l'éthoxyquine ; la lactoferrine ; la lactoperoxydase, et les dérivés nitroxydes ; les superoxyde dismutases ; le glutathion peroxydase ; les extraits végétaux à activité antiradicalaire tels que l'extrait aqueux de germe de blé commercialisé par la société Silab sous la référence Detoxiline ; et leurs mélanges.

Comme agents anti-élastase, on peut citer notamment les dérivés peptidiques, et notamment les peptides de graines de légumineuses tels que ceux commercialisés par les Laboratoires Sériobiologiques de Nancy sous la référence Parelastyl; les dérivés de N-acylamino-amides décrits dans la demande FR0007344, comme par exemple le {2-[acétyl-(3-trifluorométhyl-phényl)-amino]-3-méthyl-butyrylamino} acétate d'éthyle et l'acide {2-[acétyl-(3-trifluorométhylphényl)-amino]-3-méthyl-butyryl-amino} acétique ; et leurs mélanges. Comme agents anti-collagénase, on peut citer les inhibiteurs de métalloprotéase, tels que l'acide éthylènediamine tétracétique (EDTA), la cystéine ; et leurs mélanges.

Comme protides, on peut citer par exemple les protéines (de blé ou de soja), leurs hydrolysats, comme ceux commercialisés par la société Silab sous le référence Tensine, et leurs mélanges.

Comme dérivés d'acide gras, on peut citer notamment les phospholipides polyinsaturés dont les phospholipides d'acide gras essentiels de poulpe, et leurs mélanges.

Comme stéroïdes, on peut citer par exemple la DHEA ou déhydroépiandrostérone, ses précurseurs biologiques, ses métabolites, et leurs mélanges. Par "précurseurs biologiques" de la DHEA, on entend notamment la Δ5-prégnénolone, la 17α-hydroxy prégnénolone et le sulfate de 17α-hydroxy prégnénolone. Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA) et la 7-céto-DHEA qui est elle-même un métabolite de la 7β-hydroxy DHEA.

Comme oligo-éléments, on peut par exemple le cuivre, le zinc, le sélénium, le fer, le magnésium, le manganèse ; et leurs mélanges.

Comme agents blanchissants, on peut utiliser tout composé permettant de traiter ou de prévenir les taches de vieillissement, c'est-à-dire tout composé dépigmentant qui agit directement sur la vitalité des mélanocytes épidermiques où se déroule la mélanogénèse et/ou qui interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation. On peut citer par exemple comme actif blanchissant, l'acide kojique et ses dérivés, l'hydroquinone et ses dérivés tels que l'arbutine et ses esters ; l'acide ellagique et ses dérivés ; les extraits de plantes, et notamment de réglisse, de mûrier ou de scutellaire ; le glutathion et ses précurseurs ; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylamino-carbonyl-4-aminophénol ; et les mélanges de ces composés.

Comme extraits d'algues, on peut citer les extraits d'algues rouges ou brunes, et par exemple l'extrait d'algues brunes de la famille des Laminaires, comme les extraits de l'espèce *Laminaria digitata,* et plus particulièrement celui vendu par la société CODIF sous la dénomination PHYCOSACCHARIDES, qui est une solution concentrée d'un oligosaccharide obtenu par dépolymérisation enzymatique contrôlée de polysaccharides membranaires d'une algue brune. Il comprend l'enchaînement de deux acides uriques : acide mannuronique et l'acide guluronique.

Comme extraits de planctons, on peut citer le plancton en dispersion aqueuse (nom CTFA: Vitreoscilla Ferment) commercialisé sous la dénomination MEXORYL SAH par la société Chimex.

Comme filtres, on peut utiliser dans la composition utilisée selon l'invention tous les filtres solaires chimiques UVA et UVB ou filtres physiques habituellement utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl bènzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(tert.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolyl-benzoxazole ;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence des nano-pigments.

Comme enzymes, on peut utiliser toute enzyme d'origine animale, microbiologique (bactérienne, fongique ou virale) ou synthétique (obtenue par synthèse chimique ou biotechnologique), sous forme cristalline pure ou sous une forme diluée dans un diluant inerte. On peut citer par exemple parmi les lipases, les protéases, les phospholipases, les cellulases, les peroxydases notamment les lactoperoxydases, les catalases, les superoxyde dismutases, ou parmi des extraits végétaux contenant les enzymes précitées, et leurs mélanges.

Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines, les 2-arylbenzofuranes, et leurs mélanges. A cet égard, on se reportera avantageusement pour une revue complète sur les isoflavonoïdes, leurs méthodes d'analyse et leurs sources, au chapitre 5 "Isoflavonoïds" écrit par P.M. Dewick, dans The Flavonoïds, Harbone éditeur, pp. 125-157 (1988).

Les isoflavonoïdes peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique.

On préfère utiliser les isoflavonoïdes d'origine naturelle. Parmi ceux-ci, on peut citer : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.

Comme céramides, on peut citer par exemple la N-oléoyl-dihydrosphingosine, la N-stéaroyl phytosphingosine, le N-α-hydroxybéhénoyl-dihydrosphingosine, la N-α -hydroxypalmitoyl-dihydrosphingosine, la N-linoléoyldihydrosphingosine, la N-palmitoyldihydrosphingosine, la N-stéaroyldihydrosphingosine, la N-béhénoyldihydrosphingosine, et leurs mélanges.

Comme gélifiants, on peut citer par exemple les polymères carboxyvinyliques tels que les carbopols (carbomers) et les Pemulen (Copolymère acrylate/C₁₀C₃₀₋alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

La composition peut éventuellement contenir des charges. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

La composition de l'invention peut constituer un produit de soin de la peau (crème, lotion) et/ou de maquillage de la peau (fond de teint, fard à joues). Elle peut être utilisée notamment pour lutter et/ou prévenir le vieillissement de la peau, notamment en lissant les ridules et diminuant les rides, en éclaircissant le teint, en atténuant, voire en éliminant les taches pigmentaires apparaissant au cours du temps, en protégeant la peau des rayonnements U.V., en nourrissant la peau, en régénérant les tissus cutanés. Elle peut être utilisée aussi pour éviter le regraissage de la peau.

La composition de l'invention peut aussi servir à traiter l'acné et à traiter les blessures en favorisant la cicatrisation.

Ainsi, l'invention a encore pour objet une utilisation cosmétique de la composition définie ci-dessus pour un traitement cosmétique de la peau en vue de lisser les ridules, de diminuer les rides, d'éclaircir le teint, d'atténuer les taches pigmentaires, de lutter contre les méfaits des rayonnements U.V., d'éviter le regraissage de la peau.

L'invention a encore pour objet une utilisation de la composition définie ci-dessus pour la fabrication d'un onguent destiné à un traitement dermatologique de la peau, comme l'acné et la cicatrisation.

L'invention a encore pour objet un procédé de traitement cosmétique des signes du vieillissement de la peau, caractérisé en ce qu'il consiste à appliquer sur la peau, une composition telle définie ci-dessus.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Emulsion eau-dans-huile

| | |
|---|---|
| *Phase A :* | |
| Hydrogenated polyisobutene | 5,5 % |
| Isostearyl neopentanoate | 3,5 % |
| PEG-20 stearate | 1 % |
| Glyceryl stearate et PEG-100 stearate (Arlacel 165) | 2 % |
| Cetyl alcohol | 0,5 % |
| Stearyl alcohol | 0,5 % |
| Stearic acid | 1 % |
| *Phase A' :* | |
| Cyclomethicone | 11 % |
| Fibres de coton | 3 % |
| *Phase B :* | |
| Conservateurs | qs |
| Triéthanolamine | 0,03 % |
| Co-enzyme Q10 | 0,05 % |
| Eau | qsp 100 % |
| *Phase C :* | |
| SEPIGEL 305 | 1 % |

Mode opératoire : On chauffe la phase A sous agitation jusqu'à homogénéité. Après refroidissement on ajoute la phase A'. On chauffe la phase B sous agitation, puis on verse B dans A toujours sous agitation. Après refroidissement à 50°C on incorpore à l'émulsion la phase C.

On obtient une crème apte à estomper et traiter les rides et ridules de la peau.

### Exemple 2 : Emulsion H/E sans tensioactif

| | |
|---|---|
| *Phase huileuse* | |
| Huiles végétales | 12 % |
| Filtre UVA | 2 % |
| Filtre UVB | 4 % |
| Cyclopentasiloxane | 6 % |
| *Phase aqueuse* | |
| Co-enzyme Q10 | 0,01 % |
| EDTA disodique | 0,05 % |
| Diglycol/CHDM/Isophtalates/SIP Copolymer (Eastman AQ38S d'EASTMAN CHEMICAL) | 2 % |
| Glycérine | 5 % |
| Ethanol | 10 % |
| Fibres de polyamide | |
| (Polyamide 0.9dtex, 0,3mm - société Paul Bonte) | 2 % |
| Eau | qsp 100 % |

Mode opératoire: on prépare séparément à chaud les phases huileuse et aqueuse (sans les fibres) et on verse la phase huileuse dans la phase aqueuse sous agitation, puis on ajoute les fibres à 40°C.

On obtient une émulsion fluide permettant de traiter les signes du vieillissement de la peau en surface et en profondeur.

## Revendications

1. Composition pour application topique sur la peau, contenant des fibres choisies parmi les fibres de soie, de coton, de laine, de lin, de cellulose extraites notamment du bois, des légumes ou des algues, de polyamide, de cellulose modifiée, de poly-p-phénylène téréphtalamide, en acrylique, de polyoléfine, de verre, de silice, d'aramide, de carbone, de Téflon® , de collagène insoluble, de polyesters, de polychlorure de vinyle ou de vinylidène, d'alcool polyvinylique, de polyacrylonitrile, de chitosane, de polyéthylène phtalate, des fibres formées d'un mélange de polymères, les fibres synthétiques résorbables, et leurs mélanges, et au moins un ou plusieurs composés choisis dans le groupe des ubiquinones et de leurs dérivés.

2. Composition selon la revendication 1, **caractérisée en ce que** les fibres ont une longueur (L) allant de 1 µm à 10 mm.

3. Composition selon la revendication 1 ou 2, **caractérisée en ce que** les fibres ont une section comprise dans un cercle de diamètre (D) allant de 1 nm à 100 µm.

4. Composition selon l'une quelconque des revendications précédentes, **caractérisée en ce que** les fibres ont un facteur de forme (L/D) allant de 5 à 150.

5. Composition selon l'une quelconque des revendications précédentes, **caractérisée en ce que** les fibres ont un titre allant de 0,15 à 30 deniers.

6. Composition selon l'une quelconque des revendications précédentes, **caractérisée en ce que** les fibres sont enrobées et/ou fonctionnalisées.

7. Composition selon l'une quelconque des revendications précédentes, **caractérisée en ce que** les fibres sont choisies parmi les fibres de polyamide, les fibres de poly-p-phénylène téréphtalamide, les fibres de coton et leurs mélanges.

8. Composition selon l'une quelconque des revendications précédentes, **caractérisée en ce que** les fibres sont présentes en une quantité allant de 0,01 à 50 % en poids et de préférence de 1 à 20 % en poids par rapport au poids total de la composition.

9. Composition selon l'une quelconque des revendications précédentes, **caractérisée en ce que** l'ubiquinone est choisie parmi les co-enzymes Q, les alkyl-ubiquinones et leurs mélanges.

10. Composition selon l'une quelconque des revendications précédentes, **caractérisée en ce que** l'ubiquinone est la co-enzyme Q10.

11. Composition selon l'une quelconque des revendications précédentes, **caractérisée en ce que** la quantité d'ubiquinone(s) va de 0,0001 à 30 % en poids et de préférence de 0,01 à 20 % en poids de matière active par rapport au poids total de la composition.

12. Composition selon l'une quelconque des revendications précédentes, **caractérisée en ce qu'**elle contient en outre au moins un actif choisi parmi les vitamines, les hydratants, les actifs anti-radicaux libres, les α-hydroxy-acides, les p-hydroxy-acides, les rétinoïdes, les agents anti-élastase, les protides, les dérivés d'acide gras, les stéroïdes, les oligo-éléments, les agents blanchissants, les extraits d'algues, les planctons, les filtres solaires, les enzymes, les flavonoïdes, les céramides, et leurs mélanges.

13. Composition selon l'une quelconque des revendications précédentes, **caractérisée en ce qu'**elle comprend un milieu physiologiquement acceptable comprenant de l'eau.

14. Composition selon l'une quelconque des revendications précédentes, **caractérisée en ce qu'**elle est sous forme d'une émulsion.

15. Utilisation cosmétique de la composition cosmétique selon l'une quelconque des revendications précédentes, pour un traitement cosmétique de la peau en vue de lisser les ridules, de diminuer les rides, d'éclaircir le teint, d'atténuer les taches pigmentaires, de lutter contre les méfaits des rayonnements U.V., d'éviter le regraissage de la peau.

16. Utilisation de la composition selon l'une quelconque des revendications 1 à 14, pour la fabrication d'un onguent destiné à un traitement dermatologique de la peau.

17. Procédé de traitement cosmétique des signes du vieillissement de la peau, **caractérisé en ce qu'**il consiste à appliquer sur la peau, une composition selon l'une quelconque des revendications 1 à 14.

## Claims

1. Composition for topical application to the skin, containing fibres chosen from silk fibres, cotton fibres, wool fibres, flax fibres, cellulose fibres extracted especially from wood, vegetables or algae, polyamide fibres, modified cellulose fibres, poly-p-phenyleneterephthalamide fibres, acrylic fibres, polyolefin fibres, glass fibres, silica fibres, aramid fibres, carbon fibres, Teflon® fibres, insoluble collagen fibres, polyester fibres, polyvinyl chloride or polyvinylidene chloride fibres, polyvinyl alcohol fibres, polyacrylonitrile fibres, chitosan fibres, polyethylene phthalate fibres, fibres formed from a blend of polymers, resorbable synthetic fibres, and mixtures thereof, and at least one or more compounds chosen from the group of ubiquinones and derivatives thereof.

2. Composition according to Claim 1, **characterized in that** the fibres have a length (L) ranging from 1 µm to 10 mm.

3. Composition according to Claim 1 or 2, **characterized in that** the fibres have a cross section that is within a circle of diameter (D) ranging from 1 nm to 100 µm.

4. Composition according to any one of the preceding claims, **characterized in that** the fibres have a shape factor (L/D) ranging from 5 to 150.

5. Composition according to any one of the preceding claims, **characterized in that** the fibres have a yarn count ranging from 0.15 to 30 denier.

6. Composition according to any one of the preceding claims, **characterized in that** the fibres are coated and/or functionalized.

7. Composition according to any one of the preceding claims, **characterized in that** the fibres are chosen from polyamide fibres, poly-p-phenyleneterephthalamide fibres and cotton fibres, and mixtures thereof.

8. Composition according to any one of the preceding claims, **characterized in that** the fibres are present in an amount ranging from 0.01% to 50% by weight and preferably from 1% to 20% by weight relative to the total weight of the composition.

9. Composition according to any one of the preceding claims, **characterized in that** the ubiquinone is chosen from the coenzymes Ω and alkylubiquinones, and mixtures thereof.

10. Composition according to any one of the preceding claims, **characterized in that** the ubiquinone is coenzyme Q10.

11. Composition according to any one of the preceding claims, **characterized in that** the amount of ubiquinone(s) ranges from 0.0001% to 30% by weight and preferably from 0.01% to 20% by weight of active material relative to the total weight of the composition.

12. Composition according to any one of the preceding claims, **characterized in that** it also contains at least one active agent chosen from vitamins, moisturizers, free-radical-scavenging active agents, α-hydroxy acids, β-hydroxy acids, retinoids, anti-elastase agents, proteins, fatty acid derivatives, steroids, trace elements, bleaching agents, algal extracts, planktons, sunscreens, enzymes, flavonoids and ceramides, and mixtures thereof.

13. Composition according to any one of the preceding claims, **characterized in that** it comprises a physiologically acceptable medium comprising water.

14. Composition according to any one of the preceding claims, **characterized in that** it is in the form of an emulsion.

15. Cosmetic use of the cosmetic composition according to any one of the preceding claims, for a cosmetic treatment of the skin in order to smooth out fine lines, reduce wrinkles, lighten the complexion, efface pigmentation marks, combat the harmful effects of.UV radiation and prevent the skin from becoming greasy.

16. Use of the composition according to any one of Claims 1 to 14, for manufacturing an ointment for a dermatological treatment of the skin.

17. Cosmetic process for treating the signs of ageing of the skin, **characterized in that** it consists in applying to the skin a composition according to any one of Claims 1 to 14.

## Patentansprüche

1. Zusammensetzung zur topischen Anwendung auf die Haut, die Fasern, die unter den Seidenfasern, Baumwollfasern, Wollfasern, Leinenfasern, Cellulosefasern, die insbesondere aus Holz, Gemüse und Algen gewonnen wurden, Polyamidfasern, Fasern aus modifizierter Cellulose, Fasern aus Poly-p-phenylenterephthalamid, Acrylfasern, Polyolefinfasern, Glasfasern, Siliciumdioxidfasern, Aramidfasern, Kohlenstofffasern, Teflon® -Fasern, Faser aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyethylenphthalatfasern, Fasern, die aus einem Gemisch von Polymeren gebildet sind, resorbierbaren synthetischen Fasern und deren Gemischen ausgewählt sind, und mindestens eine oder mehrere Verbindungen enthält, die unter den Ubichinonen und deren Derivaten ausgewählt sind.

2. Zusammensetzung nach Anspruch 1, **dadurch gekennzeichnet, dass** die Fasern eine Länge L von 1 µm bis 10 mm aufweisen.

3. Zusammensetzung nach Anspruch 1 oder 2, **dadurch gekennzeichnet, dass** die Fasern einen in einem Kreis mit einem Durchmesser (D) von 1 nm bis 100 µm enthaltenden Querschnitt aufweisen.

4. Zusammensetzung nach einem der vorhergehenden Ansprüche, **dadurch gekennzeichnet, dass** die Fasern einen Formfaktor (L/D) von 5 bis 150 besitzen.

5. Zusammensetzung nach einem der vorhergehenden Ansprüche, **dadurch gekennzeichnet, dass** die Fasern einen Titer von 0,15 bis 30 Denier haben.

6. Zusammensetzung nach einem der vorhergehenden Ansprüche, **dadurch gekennzeichnet, dass** die Fasern umhüllt und/oder funktionalisiert sind.

7. Zusammensetzung nach einem der vorhergehenden Ansprüche, **dadurch gekennzeichnet, dass** die Fasern unter den Polyamidfasern, Poly-p-phenylenterephthalamidfasern, Baumwollfasern und deren Gemischen ausgewählt sind.

8. Zusammensetzung nach einem der vorhergehenden Ansprüche, **dadurch gekennzeichnet, dass** die Fasern in einer Menge von 0,01 bis 50 Gew.-% und vorzugsweise 1 bis 20 Gew.-%, bezogen auf das Gesamtgewicht der Zusammensetzung, enthalten sind.

9. Zusammensetzung nach einem der vorhergehenden Ansprüche, **dadurch gekennzeichnet, dass** das Ubichinon unter den Coenzyrnen Q, Alkylubichinonen und deren Gemischen ausgewählt ist.

10. Zusammensetzung nach einem der vorhergehenden Ansprüche, **dadurch gekennzeichnet, dass** das Ubichinon das Coenzym Q10 ist.

11. Zusammensetzung nach einem der vorhergehenden Ansprüche, **dadurch gekennzeichnet, dass** der Mengenanteil des Ubichinons oder der Ubichinone im Bereich von 0,0001 bis 30 Gew,-% und vorzugsweise 0,01 bis 20 Gew.-% wirksame Substanz, bezogen auf das Gesamtgewicht der Zusammensetzung, liegt.

12. Zusammensetzung nach einem der vorhergehenden Ansprüche, **dadurch gekennzeichnet, dass** sie ferner mindestens einen Wirkstoff enthält, der unter den Vitaminen, Hydratisierungsmitteln, Radikalfängern für freie Radikale, α-Hydroxysäuren, β-Hydroxysäuren, Retinoiden, Anti-Elastase-Stoffen, Protiden, Fettsäurederivaten, Steroiden, Spurenelementen, Bleichmitteln, Algenextrakten, Plakton, Sonnenschutzfiltern, Enzymen, Flavonoiden, Ceramiden und deren Gemischen ausgewählt ist.

13. Zusammensetzung nach einem der vorhergehenden Ansprüche, **dadurch gekennzeichnet, dass** sie ferner ein physiologisch akzeptables Medium enthält, das Wasser umfasst.

14. Zusammensetzung nach einem der vorhergehenden Ansprüche, **dadurch gekennzeichnet, dass** sie als Emulsion vorliegt.

15. Kosmetische Verwendung der kosmetischen Zusammensetzung nach einem der vorhergehenden Ansprüche für die kosmetische Behandlung der Haut, um Fältchen zu glätten, Falten zu mildern, den Teint strahlender erscheinen zu lassen, Pigmentflekken zu lindern, die schädlichen Wirkungen der UV-Strahlung zu bekämpfen und die Rückfettung der Haut zu verhindern.

16. Verwendung der Zusammensetzung nach einem der Ansprüche 1 bis 14 für die Herstellung einer Salbe, die für eine dermatologische Behandlung der Haut vorgesehen ist.

17. Verfahren zur kosmetischen Behandlung der Anzeichen der Hautalterung, **dadurch gekennzeichnet, dass** es darin besteht, eine Zusammensetzung nach einem der Ansprüche 1 bis 14 auf die Haut aufzutragen.
